# EUROPEAN PATENT APPLICATION

(11) **EP 3 123 924 A1**
(43) Date of publication of application: **01.02.2017**
(21) Application number: 14887647.7
(22) Date of filing: 12.11.2014
(51) Int. Cl.: A61B 1/04, G02B 23/24

(54) **ENDOSCOPIC IMAGING DEVICE**

(30) Priority: 28.03.2014 JP 2014068517
(71) Applicant: Fujifilm Corporation, Minato-ku Tokyo 106-8620 (JP)
(72) Inventor: YANO Takashi, Ashigarakami-gun Kanagawa 258-8538 (JP)
(74) Representative: Klunker . Schmitt-Nilson . Hirsch
(86) International application number: PCT/JP2014/079971
(87) International publication number: WO 2015/145859

(57) **Abstract**

An imaging device, which is disposed in a tip portion of the endoscope, includes: a lens barrel that holds an objective optical system receiving image light of a portion to be observed and forming an image; a rectangular prism changing the direction of a light path of image light, which has passed through the objective optical system, at a right angle; an imaging element of which an imaging surface is disposed along a plane orthogonal to the direction of the light path changed by the rectangular prism; and a board on which the imaging element is mounted. The board includes a tip-side region that extends toward a tip side from an imaging element mounting region in which the imaging element is mounted, and an electronic component of a drive circuit necessary to drive the imaging element is mounted in the tip-side region.

## Description

### BACKGROUND OF THE INVENTION

### 1. Field of the Invention

The present invention relates to an endoscopic imaging device, and more particularly, to an endoscopic imaging device mounted on an imaging section of an endoscope, which takes an image of a portion to be observed and is provided at a tip portion or the like of an endoscope insertion part of a flexible scope, and contributing to the reduction of the size of the imaging section of the endoscope.

### 2. Description of the Related Art

An endoscopic imaging device is a device that is mounted in a tip portion of a long insertion part, which is to be inserted into a body cavity, of an endoscope (hereinafter, the insertion part and the tip portion of the endoscope will be referred to as an endoscope insertion part and an endoscope tip portion) and takes an image of the inside of the body cavity. The endoscopic imaging device includes: an objective optical system that forms an image by using image light emitted from a portion to be observed by an endoscope; and a solid-state imaging element, such as a charge coupled device (CCD) type image sensor or a complementary metal oxide semiconductor (CMOS) type image sensor, converting a light image, which is formed by the objective optical system, to an imaging signal that is an electrical signal.

When an imaging surface of the solid-state imaging element is disposed so as to be orthogonal to an optical axis of the objective optical system in the endoscopic imaging device, it is necessary to ensure a space, in which a board on which the solid-state imaging element and electronic components of a peripheral circuit are mounted is disposed, in a direction (a radial direction) orthogonal to the optical axis of the objective optical system. For this reason, the diameter of the endoscope tip portion is increased. Accordingly, an endoscopic imaging device of which an imaging surface of a solid-state imaging element is disposed parallel to an optical axis of an objective optical system as disclosed in JP2012-217605A has been known in the past.

According to the endoscopic imaging device, a solid-state imaging element is disposed closer to a base end than the objective optical system, of which an optical axis is disposed along the axial direction of the endoscope tip portion (the axial direction of the endoscope insertion part), with a rectangular prism interposed between the objective optical system and itself. Further, a light path of image light, which has passed through the objective optical system and is emitted from a portion to be observed, is bent at a right angle by the rectangular prism, and the imaging surface of the solid-state imaging element is disposed along a direction orthogonal to the bent light path. Accordingly, since the solid-state imaging element and the board on which the electronic components of the peripheral circuit are mounted are disposed parallel to the optical axis of the objective optical system, the diameter of the endoscope tip portion is reduced.

Furthermore, JP2010-69217A discloses an endoscopic imaging device of which an imaging surface of a solid-state imaging element is disposed so as to be orthogonal to an optical axis of an objective optical system. In the endoscopic imaging device, a board on which the solid-state imaging element is mounted is bent on a tip side thereof (an objective optical system side thereof) and is in contact with a lens barrel of the objective optical system and the electronic components of the peripheral circuit are mounted on the bent portion of the board, so that heat generated in the solid-state imaging element or the peripheral circuit is radiated to the lens barrel and space-saving is achieved.

### SUMMARY OF THE INVENTION

Since the imaging element is disposed along the direction orthogonal to the axis of the endoscope tip portion in the configuration of the endoscopic imaging device of JP2010-69217A, the diameter of the endoscope tip portion needs to be set so that the horizontal and vertical size of the solid-state imaging element is included in a circle having the diameter of the endoscope tip portion. Particularly, since a CMOS solid-state imaging element has an advantage that a part of a peripheral circuit, such as a signal processing circuit, can be embedded in the form of an on-chip, the size of the CMOS solid-state imaging element is increased to that extent. Accordingly, there is a limitation in reducing the diameter of the endoscope tip portion even though the configuration disclosed in JP2010-69217A is employed.

In this regard, it is possible to set the diameter of the endoscope tip portion to the minimum size, in which the solid-state imaging element is included, by disposing the solid-state imaging element and the board near the axis of the endoscope tip portion in a case in which the solid-state imaging element and the board are disposed parallel to the optical axis of the objective optical system as in JP2012-217605A. Accordingly, the configuration of JP2012-217605A is more advantageous than the configuration of JP2010-69217A in terms of the reduction of the diameter of the endoscope tip portion.

However, there is a drawback that the length of the endoscope tip portion in the axial direction is made to be longer than that of the structure of JP2012-217605A in order to dispose the board so that the board is parallel to the optical axis of the objective optical system even in the case of the structure of the endoscopic imaging device of JP2012-217605A. For example, a bendable portion, which is bent vertically and laterally by an operator's operation, is provided in the case of a general flexible scope so that the direction of observation performed by the endoscopic imaging device can be changed further at the base end than at the endoscope tip portion of the endoscope insertion part. When the orientation of the endoscope tip portion is changed by changing of the bending angle of the bendable portion, the amplitude of the tip of the endoscope tip portion where the objective optical system is disposed is increased as the length of the endoscope tip portion is increased. For this reason, it is difficult to adjust the direction of observation and a gap in the body cavity, which is necessary to change the orientation of the endoscope tip portion, is also increased. Accordingly, it is preferable that not only the size of the endoscope tip portion in the radial direction but also the size of the endoscope tip portion in the axial direction is small, and it is preferable that the size of the endoscopic imaging device of JP2012-217605A in the axial direction is also reduced. That is, it is preferable that the size of the endoscopic imaging device in a direction parallel to the optical axis of the objective optical system is reduced (the length of the endoscopic imaging device is reduced).

The invention has been made in consideration of the above-mentioned circumstances, and an object of the invention is to provide an endoscopic imaging device of which the size in a direction parallel to an optical axis of an objective optical system is reduced and which can contribute to the reduction of the size of an imaging section of an endoscope.

In order to achieve the object, an endoscopic imaging device according to an aspect of the invention comprises: an objective optical system that receives image light of a portion to be observed and forms an image; a reflector changing a direction of a light path of image light, which has passed through the objective optical system, to one direction; a solid-state imaging element of which an imaging surface is disposed along a plane orthogonal to the direction of the light path changed by the reflector and which takes a light image formed on the imaging surface; and a board where the solid-state imaging element and an electronic component necessary to drive the solid-state imaging element are mounted and the electronic component is mounted in a region extending toward the objective optical system from a region in which the solid-state imaging element is mounted.

According to this aspect, the electronic component necessary to drive the solid-state imaging element is mounted in a region of the board that extends toward the tip side (the objective optical system) from the region, on which the solid-state imaging element is mounted, in the direction parallel to the optical axis of the objective optical system. Accordingly, it is possible to reduce the length of a portion of the board that extends toward the base end (the side opposite to the objective optical system) from the region in which the solid-state imaging element is mounted. Therefore, since it is possible to reduce the length of the endoscopic imaging device in the direction parallel to the optical axis of the objective optical system, it is possible to contribute to the reduction of the size of the imaging section of the endoscope on which the endoscopic imaging device of the aspect is mounted.

In the endoscopic imaging device according to the aspect of the invention, the reflector may change the direction of the light path of the image light, which has passed through the objective optical system, at a right angle, and the imaging surface may be disposed parallel to an optical axis of the objective optical system.

According to this aspect, the endoscopic imaging device is adapted to be suitable for being reduced in size in the radial direction orthogonal to the optical axis of the objective optical system.

In the endoscopic imaging device according to the aspect of the invention, the solid-state imaging element and the electronic component may be mounted on the same surface of the board.

According to this aspect, in comparison with a case in which the solid-state imaging element and the electronic component are disposed on different surfaces of the board, it is advantageous for reducing a space and it is also possible to reduce a wiring distance between the solid-state imaging element and the electronic component.

In the endoscopic imaging device according to the aspect of the invention, a region of the board in which the solid-state imaging element is mounted, and a region of the board in which the electronic component is mounted may be provided in a range of a flat plate-shaped region of the board that is parallel to the imaging surface.

According to this aspect, special work, such as the bending of the board, is not required and it is advantageous in reducing the size of the endoscopic imaging device in the radial direction orthogonal to the optical axis of the objective optical system.

In the endoscopic imaging device according to the aspect of the invention, the objective optical system may include a plurality of lenses, the plurality of lenses may be housed and held in a lens barrel, and the electronic component mounted on the board may be disposed in a gap between the lens barrel and the board.

According to this aspect, a dead space between the board and the lens barrel is effectively utilized so that the electronic component is disposed in the region of the board closer to the tip side than the region on which the imaging element is mounted. Accordingly, it is possible to prevent the size of the endoscopic imaging device from being increased in the radial direction orthogonal to the optical axis of the objective optical system.

In the endoscopic imaging device according to the aspect of the invention, the reflector may be a rectangular prism; one surface of two surfaces of the rectangular prism between which a right angle is formed may be an incident surface, and the other surface thereof may be a light-emitting surface; the image light, which is emitted from the objective optical system and incident from the incident surface and emitted from the objective optical system, may be reflected by an inclined surface of the rectangular prism and emitted from the light-emitting surface; the solid-state imaging element may be provided with a cover glass that covers the imaging surface; the light-emitting surface of the rectangular prism may be joined to a part of a region of a surface of the cover glass; and the electronic component may be disposed by using the gap that is formed so as to correspond to a thickness of the cover glass and the solid-state imaging element in a region protruding toward the objective optical system from the region, to which the light-emitting surface of the rectangular prism is joined, of the surface of the cover glass.

According to this aspect, in a case in which the solid-state imaging element is provided with the cover glass, a gap corresponding to the thickness of the imaging element and the cover glass is formed as a dead space between the board and the lens barrel. Accordingly, it is possible to prevent the size of the endoscopic imaging device from being increased in the radial direction, which is orthogonal to the optical axis of the objective optical system, by effectively utilizing the gap to dispose the electronic component.

In the endoscopic imaging device according to the aspect of the invention, the solid-state imaging element may be an MOS type imaging element, and the electronic component may be a vibrator or an oscillator that generates a clock signal of the solid-state imaging element.

According to this aspect, in a case in which an MOS type imaging element is used as the solid-state imaging element, a vibrator or an oscillator, which generates a clock signal, is mounted in a region of the board, which is closer to the tip side than the solid-state imaging element, as the electronic component that is necessary to drive the solid-state imaging element. Since electronic components of other circuits, such as peripheral circuits relating to the input and output of signals of the solid-state imaging element, do not need to be disposed in that region, it is possible to achieve an ideal disposition in which a vibrator or an oscillator is mounted near the solid-state imaging element.

In the endoscopic imaging device according to the aspect of the invention, the objective optical system, the reflector, the solid-state imaging element, and the board may be mounted in a tip portion of an endoscope insertion part to be inserted into a subject.

According to this aspect, it is possible to reduce the size of the tip portion of the endoscope insertion part, that is, the endoscope tip portion. The reduction of the size of the endoscopic imaging device contributes to the reduction of the size of the imaging section of the endoscope. The reduction of the size of the imaging section of the endoscope is not limited to the flexible scope in which the endoscope tip portion is disposed on the tip side of the bendable portion, and is effective in various kinds of endoscope.

According to the invention, it is possible to reduce the size of the endoscopic imaging device in a direction parallel to an optical axis of an objective optical system, and to contribute to the reduction of the size of an imaging section of an endoscope.

### BRIEF DESCRIPTION OF THE DRAWINGS

Fig. 1 is a view showing the entire structure of an endoscope that employs an endoscopic imaging device to which the invention is applied.
Fig. 2 is a perspective view showing the appearance of an endoscope tip portion of Fig. 1.
Fig. 3 is a view showing the structure of the endoscopic imaging device of a first embodiment in a section taken along line A-A of Fig. 2.
Fig. 4 is a view showing components of the endoscopic imaging device of the first embodiment.
Fig. 5 is a view showing the structure of an endoscopic imaging device of a comparative example in the section taken along line A-A of Fig. 2.
Fig. 6 is a view showing components of the endoscopic imaging device of the comparative example.
Fig. 7 is a view showing the structure of an endoscopic imaging device of a second embodiment in the section taken along line A-A of Fig. 2.
Fig. 8 is a view showing components of the endoscopic imaging device of the second embodiment.

### DESCRIPTION OF THE PREFERRED EMBODIMENTS

Preferred embodiments of the invention will be described in detail below with reference to the accompanying drawings.

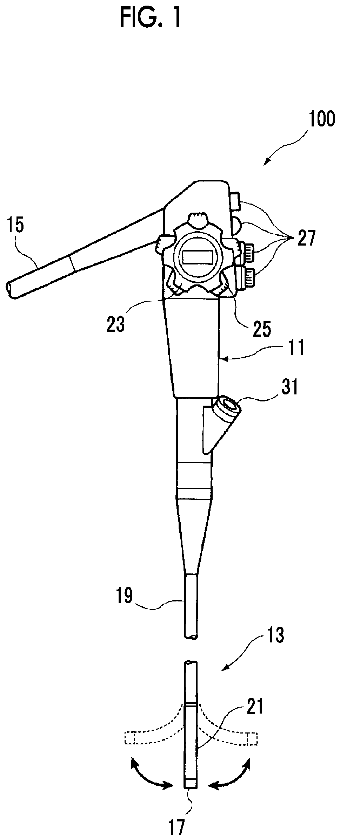

Fig. 1 is a view showing the entire structure of an endoscope that employs an endoscopic imaging device to which the invention is applied.

An endoscope 100 of Fig. 1 is a well-known flexible scope, and includes a body operation unit 11 and an endoscope insertion part 13 that is installed consecutively to the body operation unit 11 and is inserted into a body cavity.

A universal cord 15 is connected to the body operation unit 11, and a tip of the universal cord 15 is connected to a light source device, which supplies illumination light to the endoscope 100, and a processor device, which performs processing and the like of an image obtained from the endoscope 100, through a connector (not shown). The connector of the universal cord 15 has a structure that varies according to whether the processor device and the light source device are formed as an integrated device or separate devices, and the like.

The endoscope insertion part 13 includes a soft portion 19, a bendable portion 21, and a hard tip portion 17 in this order toward a tip side thereof from the body operation unit 11 (a base end thereof). The soft portion 19 has flexibility, and the bendable portion 21 can be bent in an intended direction. The bendable portion 21 is remotely operated so as to be bent by the rotation of angle knobs 23 and 25 of the body operation unit 11. Accordingly, the tip portion 17 can be directed in a desired direction. Further, an endoscopic imaging device to which the invention is applied is mounted on the tip portion 17 as described below.

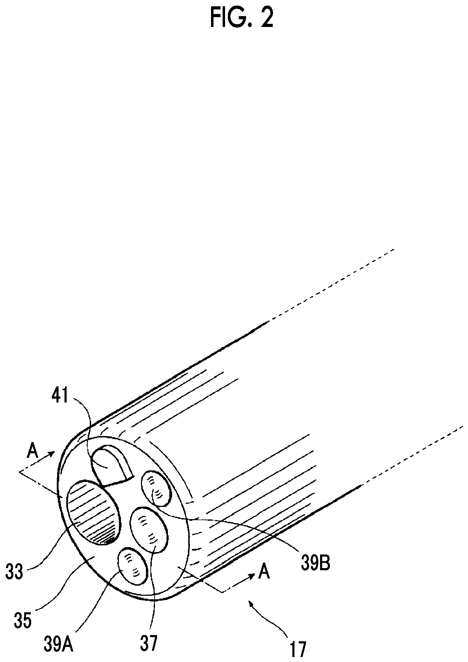

The body operation unit 11 is provided with various buttons 27, such as an air supply/water supply button, a suction button, and a shutter button, in addition to the angle knobs 23 and 25. Furthermore, a forceps insertion part 31 is provided on the tip side of the body operation unit 11. A treatment tool, such as forceps, is inserted into the forceps insertion part 31. The treatment tool inserted from the forceps insertion part 31 is inserted into a forceps channel (a forceps pipe line), which is formed in the endoscope insertion part 13, and is guided to the outside from a forceps port 33 (see Fig. 2) that is formed in the tip portion 17 of the endoscope insertion part 13.

Fig. 2 is a perspective view showing the appearance of the tip portion 17 of the endoscope insertion part 13.

As shown in Fig. 2, the tip portion 17, which is the tip portion of the endoscope insertion part 13, (hereinafter, referred to as an endoscope tip portion 17) includes an observation window 37, illumination windows 39A and 39B, the forceps port 33, and an air supply/water supply nozzle 41 that are formed at an end face 35 thereof.

The observation window 37 forms a part of an objective optical system of the endoscopic imaging device, and allows image light, which is sent from a portion to be observed, to enter the objective optical system. The light image of the portion to be observed, which is formed by the image light having entered the objective optical system, is converted to an imaging signal, which is an electrical signal, by a solid-state imaging element to be described below, and is transmitted to the processor device, which is connected to the endoscope 100, through a signal cable that is provided in the endoscope 100.

The illumination windows 39A and 39B emit illumination light, which is transmitted through a light guide provided in the endoscope 100, to the portion to be observed. The illumination light, which is transmitted through the light guide, is supplied from the light source device that is connected to the endoscope 100.

Since the air supply/water supply nozzle 41 is disposed so that an ejection port of the air supply/water supply nozzle 41 faces the observation window 37, the air supply/water supply nozzle 41 supplies air or water to the observation window 37 according to the operation of the air supply/water supply button of the body operation unit 11.

The forceps port 33 communicates with the forceps insertion part 31, which is provided on the tip side of the body operation unit 11, through the forceps channel provided in the endoscope 100, and guides the treatment tool that is inserted from the forceps insertion part 31 as described above.

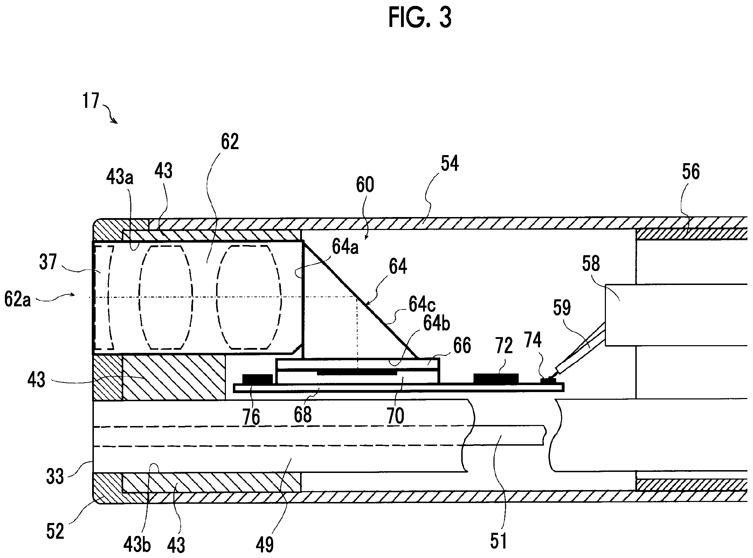

Fig. 3 is a view showing the structure of the endoscopic imaging device of a first embodiment in a section taken along line A-A of Fig. 2. The schematic view of the endoscopic imaging device, which is not viewed in the section and is viewed from a side, is shown in Fig. 3.

As shown in Fig. 3, the endoscope tip portion 17 includes a tip portion body 43 that is made of a hard material, such as a stainless steel material, as a frame where various members are assembled. A metal sleeve (not shown) is connected to the outer periphery of a base end portion of the tip portion body 43. Among a plurality of nodal rings that are connected to the bendable portion 21 (see Fig. 1), a nodal ring 56 disposed at the tip is connected to the metal sleeve. Further, the outer periphery of the metal sleeve is covered with an outer tube 54 that extends up to the outer periphery of the tip portion body 43. The outer periphery of a tip portion of the tip portion body 43 is covered with a tip cover 52 that is joined to the tip of the outer tube 54.

The tip portion body 43 includes holes 43a and 43b passing through the tip portion body 43 in the axial direction of the endoscope tip portion 17 that is also the axial direction of the endoscope insertion part 13.

A lens barrel 62 of an imaging device 60, which is the endoscopic imaging device of the first embodiment, is fitted and fixed to the hole 43a and the observation window 37, which is provided at the foremost portion of an objective optical system 62a housed and held in the lens barrel 62, is disposed at the position of the end face 35 as shown in Fig. 2.

A forceps pipe 49, which is a tip portion of the pipe line connected from the forceps insertion part 31 as the forceps channel, is fitted and fixed to the hole 43b, and a tip opening of the forceps pipe 49 is disposed at the end face 35 as the forceps port 33 of Fig. 2.

Further, an air supply/water supply pipe 51, which is connected to the air supply/water supply nozzle 41 shown in Fig. 2, and a member, such as the light guide (not shown) transmitting illumination light to the illumination windows 39A and 39B, are fixed to the tip portion body 43.

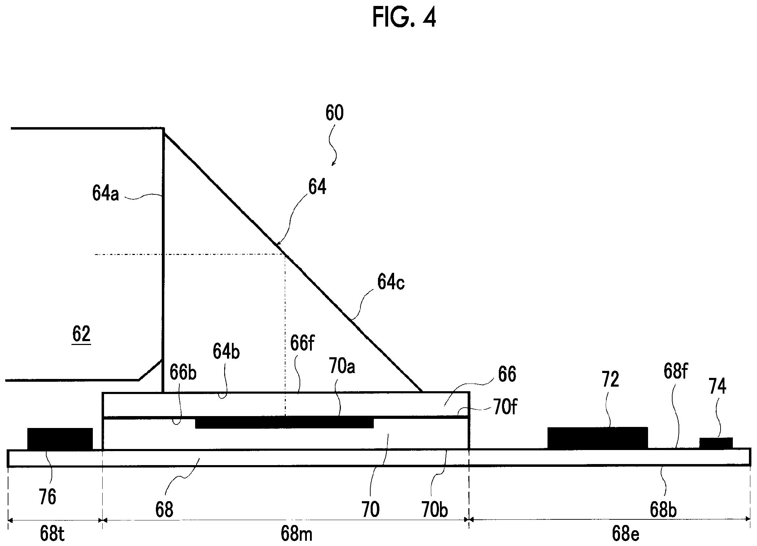

Next, the structure of the imaging device 60, which is the endoscopic imaging device of the first embodiment, will be described. Fig. 4 is a view showing only components of the imaging device 60 in the endoscope tip portion 17 of Fig. 3, and is a view in which the tip side of the lens barrel 62 of the imaging device 60 is not shown.

As shown in Figs. 3 and 4, the imaging device 60 includes the lens barrel 62, a rectangular prism 64, a solid-state imaging element 70 (hereinafter, referred to as an imaging element 70), a board 68, and the like.

The lens barrel 62 receives and holds a plurality of lenses forming the objective optical system 62a that receives image light of the portion to be observed and forms an image, and is fitted and fixed to the hole 43a of the tip portion body 43 as described above. The observation window 37 is also included in the objective optical system 62a.

The rectangular prism 64 is a form of a reflector that changes the direction of a light path of the image light, which has passed through the objective optical system of the lens barrel 62, to one direction, and bends the light path of the image light, which has passed through the objective optical system, at a right angle.

That is, the rectangular prism 64 includes an incident surface 64a and a light-emitting surface 64b (two surfaces of the rectangular prism 64 between which a right angle is interposed) that are disposed along two planes intersecting with each other at a right angle as well known, and an inclined surface 64c that is provided along a plane intersecting with both the incident surface 64a and the light-emitting surface 64b at 45 degrees.

The incident surface 64a is fixed to a base end of the lens barrel 62, and is disposed along a plane that is orthogonal to an optical axis of the objective optical system 62a. At this time, the light-emitting surface 64b is disposed along a plane that is parallel to the optical axis of the objective optical system.

Accordingly, the image light, which has passed through the objective optical system 62a and emitted from the portion to be observed, is totally reflected by the inclined surface 64c after being incident on the rectangular prism 64 from the incident surface 64a. Accordingly, the direction of the light path of the image light is changed at right angle. Further, the image light, which is reflected by the inclined surface 64c, is emitted from the light-emitting surface 64b.

The rectangular prism 64 may not be used and other optical components, which bend a light path, such as a plane mirror, may be used as the reflector. Furthermore, the reflector may be change the direction of the light path to one direction without bending the light path of the image light, which has passed through the objective optical system, at a right angle.

The imaging element 70 is imaging means for taking a light image, which is formed on an imaging surface 70a, by the operation of the objective optical system 62a, and is, for example, a metal oxide semiconductor (MOS) type solid-state imaging element, such as a complementary metal oxide semiconductor (CMOS). The imaging element 70 is formed in the form of a rectangular plate having two surfaces, that is, a surface and a back (a surface 70f and a back 70b), and an imaging surface 70a (an imaging region) on which unit pixels including photodiodes as photoelectric conversion elements are two-dimensionally arranged in the form of a matrix is disposed on a part of the surface 70f that faces the rectangular prism 64. Further, a rectangular plate-shaped cover glass 66 is installed on the entire surface 70f. Accordingly, the imaging surface 70a is covered with the cover glass 66.

The imaging element 70 is a so-called backside irradiation type CMOS imaging element where photodiodes forming the imaging surface 70a are disposed on the light incident side rather than a circuit portion, but may be a surface irradiation type CMOS imaging element and may be a charge coupled device (CCD) imaging element. Further, the cover glass 66 is not limited to a glass material and may be made of other materials, such as a transparent resin. Furthermore, in this specification, the surface on which the image light is incident of two surfaces, that is, the surface and back of the imaging element 70 is referred to as the surface 70f and the side thereof opposite to the surface 70f is referred to as the back 70b, and the surface 70f and the back 70b do not necessarily coincide with a surface and a back that are mentioned when a backside irradiation type and the like and a solid-state imaging element are mentioned.

The light-emitting surface 64b of the rectangular prism 64 is joined to a surface 66f, which is a side on which the rectangular prism 64 is disposed (a side on which the image light is incident), of two surfaces, that is, a surface and a back of the cover glass 66 (a surface 66f and a back 66b) at a position facing the imaging surface 70a of the imaging element 70. Accordingly, the imaging surface 70a of the imaging element 70 is disposed along a plane orthogonal to the direction of the light path of the image light that is changed by the rectangular prism 64. That is, the imaging surface 70a and the surface 70f of the imaging element 70 are disposed parallel to the optical axis of the objective optical system 62a.

Accordingly, image light emitted from the light-emitting surface 64b of the rectangular prism 64 passes through the cover glass 66 and is incident on the imaging surface 70a of the imaging element 70, and the light image of the portion to be observed is formed on the imaging surface 70a by the image forming operation of the objective optical system 62a. The imaging element 70 takes the light image of the imaging surface 70a, that is, converts the light image of the imaging surface 70a to an electrical signal and outputs the electrical signal as an imaging signal.

A board 68, which is a circuit board, is disposed on the back 70b of the imaging element 70, and the imaging element 70 is mounted on the board 68. That is, the back 70b of the imaging element 70 is fixed to a surface 68f on which the rectangular prism 64 is disposed of two surfaces, that is, a surface and a back of the board 68 (a surface 68f and a back 68b). Accordingly, various connection terminals (electrode pads and the like), which are provided on the back 70b of the imaging element 70, are electrically connected to connection terminals (electrode pads and the like) of the surface 68f of the board 68 corresponding to the various connection terminals.

The board 68 is a circuit board which includes a wiring pattern for wiring the imaging element 70 and other electronic components to be mounted and on which predetermined circuits are formed, and is formed of, for example, a hard and rigid board, such as a ceramic board, in the shape of a rectangular plate that is flat as a whole. However, although not shown in the drawings, gaps formed at the peripheral portion of the board 68 and gaps installed consecutively to the gaps are filled with a sealing resin so that the board 68 is fixed without being moved. Accordingly, the board 68 may not be a rigid board and may be a flexible board (FPC: Flexible printed circuits), and is not limited to a specific type of board.

Further, the board 68 includes a base end-side region 68e that extends toward the base end of the endoscope tip portion 17 (the base end of the endoscope insertion part 13) from an imaging element mounting region 68m in which the imaging element 70 is mounted, and a tip-side region 68t that extends toward the tip (the lens barrel 62 provided at the tip of the endoscope tip portion 17).

Among peripheral circuits that are necessary to drive the imaging element 70 and to input and output signals, a peripheral circuit 72, which is a circuit necessary to input and output signals, other than a circuit (a drive circuit 76 to be described below) provided in the tip-side region 68t is disposed on a surface 68f, on which the imaging element 70 is mounted, in the base end-side region 68e of the board 68. Then, an electronic component of the peripheral circuit 72 is mounted on the board.

Further, a plurality of input and output terminals 74 (electrode pads and the like), which input and output signals to and from the circuits of the board 68, are provided on the surface 68f in the base end-side region 68e, and each signal wire 59 of a signal cable 58 is connected to each input and output terminal 74 by soldering or the like (see Fig. 3). Since the signal cable 58 is disposed in the endoscope 100 so as to be inserted into a connector to be connected to the processor device, each signal wire is connected to the processor device through the connector. Accordingly, signals are transmitted between the circuits provided on the board 68 and the processor device, so that an imaging signal output from the imaging element 70 is transmitted to the processor device through the peripheral circuit 72.

The drive circuit 76, which is a part of or all the circuits necessary to drive the imaging element 70, is disposed on the surface 68f, on which the imaging element 70 is mounted, in the tip-side region 68t of the board 68, and an electronic component of the drive circuit 76 is mounted on the surface 68f in the tip-side region 68t. That is, the imaging element 70 and the electronic component of the drive circuit 76 are mounted on the same surface of the board 68, and the electronic component of the drive circuit 76 is mounted closer to the tip side than the imaging element 70.

For example, a vibrator (crystal vibrator) or an oscillator (crystal oscillator), which generates clock signals having a predetermined frequency for the synchronization of an internal circuit of the imaging element 70, is disposed as an electronic component that forms the drive circuit 76. A vibrator (a component in which an electrode is attached to a crystal vibrator piece and which is enclosed in a package) is mounted in the tip-side region 68t of the board 68 as an electronic component of the drive circuit 76 in a case in which the imaging element 70 comprises an oscillation circuit for generating a clock other than a vibrator therein; and an oscillator, which includes an oscillation circuit housed in a package, is mounted in the tip-side region 68t of the board 68 as an electronic component of the drive circuit 76 in a case in which the imaging element 70 does not comprise an oscillation circuit for generating a clock therein.

According to the imaging device 60 of the above-mentioned first embodiment, since the drive circuit 76, which is a part of or all the circuits necessary to drive the imaging element 70, is disposed in the tip-side region 68t of the board 68, it is possible to reduce the size of the base end-side region 68e to that extent. The length of the imaging device 60 in the direction parallel to the optical axis of the objective optical system 62a can be reduced. Accordingly, it is possible to reduce the size of the endoscope tip portion 17, particularly, to reduce the length of the endoscope tip portion 17 in the axial direction.

Further, since a space in which the tip-side region 68t of the board 68 and the drive circuit 76 are disposed effectively utilizes a dead space without requiring changing the disposition of other components held at the tip portion body 43, an increase in the size of the endoscopic imaging device in a radial direction orthogonal to the optical axis of the objective optical system 62a is not caused and the diameter of the endoscope tip portion 17 is also not increased.

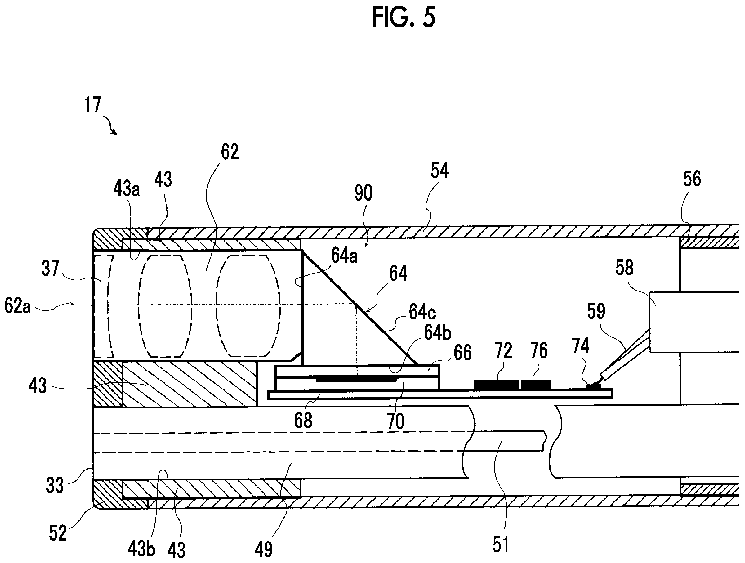

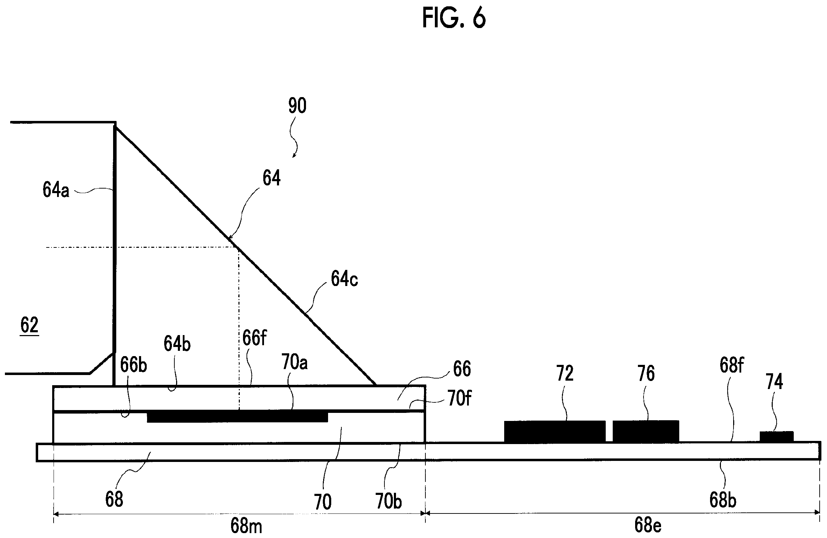

These effects will be described in detail. Fig. 5 is a view showing the structure of an imaging device 90, which is an endoscopic imaging device of a comparative example to which the invention is not applied, in the section taken along line A-A of Fig. 2 and Fig. 6 is a view showing only components of the imaging device 90 of the comparative example. Figs. 5 and 6 are views corresponding to Figs. 3 and 4, respectively; and components, which are the same as or similar to the components of the first embodiment shown in Figs. 3 and 4, will be denoted by the same reference numerals and the description thereof will be omitted.

As shown in Figs. 5 and 6, a board 68 of the imaging device 90 of the comparative example does not include a portion corresponding to the tip-side region 68t that extends toward the tip side from the imaging element mounting region 68m and on which the electronic component of the drive circuit 76 is mounted as in the case of the board 68 of the imaging device 60 of the first embodiment shown in Figs. 3 and 4. The drive circuit 76, which has been disposed in the tip-side region 68t in the imaging device 60 of the first embodiment, is disposed in a base end-side region 68e in the imaging device 90 of the comparative example. For this reason, since a space is required in the base end-side region 68e of the board 68 as that much in the imaging device 90 of the comparative example, the length of the base end-side region 68e in the optical axis of the objective optical system 62a is increased. Accordingly, the length of the endoscope tip portion 17 in the axial direction is also increased.

In contrast, since the board 68 is provided in the tip-side region 68t that protrudes toward the tip side (the lens barrel 62) from the imaging element mounting region 68m and the drive circuit 76 is disposed in the tip-side region 68t in the imaging device 60 of the first embodiment shown in Figs. 3 and 4, it is possible to reduce the length of the base end-side region 68e in the axial direction to that extent. Accordingly, it is also possible to make the length of the endoscope tip portion 17 in the axial direction be shorter than the length of the endoscope tip portion 17 where the imaging device 90 of the comparative example is mounted.

Further, a region, in which the tip-side region 68t of the board 68 is disposed, of the endoscope tip portion 17 where the imaging device 60 of the first embodiment is mounted becomes a dead space that cannot be effectively utilized to avoid the interference between the imaging device 90 and components (a forceps pipe 49 and the like) other than the imaging device 90 in the endoscope tip portion 17 in which the imaging device 90 of the comparative example is mounted as shown in Fig. 5. Furthermore, in the imaging device 60 of the first embodiment, the electronic component of the drive circuit 76 mounted in the tip-side region 68t of the board 68 is disposed in a gap between the tip-side region 68t of the board 68 and the lens barrel 62 as shown in Figs. 3 and 4. However, the gap is a gap that is generated when a gap between the board 68 and the lens barrel 62 of the imaging device 90 of the comparative example shown in Figs. 5 and 6 is not increased in size and the board 68 extends to the tip side from the imaging element mounting region 68m.

That is, for the purpose of the protection of the entire imaging element 70 and the like, the cover glass 66 is provided in the imaging element 70 used in the first embodiment so as to cover not only the area of the imaging surface 70a but also the entire surface 70f of the imaging element 70. Further, the light-emitting surface 64b of the rectangular prism 64 is joined to the surface 66f of the cover glass 66, and the cover glass 66 is present so as to protrude toward the tip side from a region to which the rectangular prism 64 is joined, and reaches the area of the side portion of the lens barrel 62. For this reason, a gap, which corresponds to the thickness of the cover glass 66 and the imaging element 70, needs to be formed between the board 68 and the lens barrel 62 so that the cover glass 66 does not interfere with the lens barrel 62. Even in the imaging device 90 of the comparative example, a gap, which corresponds to the thickness of the cover glass 66 and the imaging element 70, needs to be formed between the board 68 and the lens barrel 62 as shown in Figs. 5 and 6 in a case in which the same imaging element 70 as described above is used.

Accordingly, in a case in which the board 68 includes the tip-side region 68t as in the case of the solid-state imaging element 70 of the first embodiment, a relatively large gap, which corresponds to the thickness of the cover glass 66 and the imaging element 70, is formed between the tip-side region 68t of the board 68 and the lens barrel 62 even though the change causing an increase in the diameter of the endoscope tip portion 17, which allows an increase in the size of the gap between the board 68 and the lens barrel 62 in the imaging device 90 of the comparative example, is not added. Since the electronic component of the drive circuit 76 is disposed by effectively using the gap in the solid-state imaging element 70 of the first embodiment, the size of the endoscopic imaging device in the radial direction orthogonal to the optical axis of the objective optical system 62a is not increased and an increase in the diameter of the endoscope tip portion 17 is also not caused.

The minimum gap between the surface 68f of the tip-side region 68t of the board 68 of the solid-state imaging element 70 of the first embodiment and the surface of the lens barrel 62 varies according to the types of the cover glass 66 and the imaging element 70. However, since the sum of the thickness of the cover glass 66 and the thickness of the imaging element 70 is in the range of about 0.5 mm to about 0.7 mm, the minimum gap is equal to or larger than the sum of the thickness of the cover glass 66 and the thickness of the imaging element 70.

The electronic component of the drive circuit 76 varies according to the type of the drive circuit 76. If the electronic component of the drive circuit 76 is a crystal vibrator or a crystal oscillator as described above, the electronic component of the drive circuit 76 can be disposed in the gap between the tip-side region 68t and the lens barrel 62.

Moreover, according to the imaging device 60 of the first embodiment, there is an advantage of disposing the drive circuit 76 at a position very close to the imaging element 70. Particularly, in a case in which the electronic component of the drive circuit 76 is a crystal vibrator or a crystal oscillator, the disposition of the crystal vibrator or the crystal oscillator at a position close to the imaging element 70 is also beneficial to the prevention of the generation of noises. There is a case in which the drive circuit 76 cannot be disposed close to the imaging element 70 due to the disposition of other circuits in the imaging device 90 of the comparative example of Figs. 5 and 6. However, since only the drive circuit 76 is present in the tip-side region 68t of the board 68 in the case of the imaging device 60 of the first embodiment, the drive circuit 76 can be disposed near the imaging element 70.

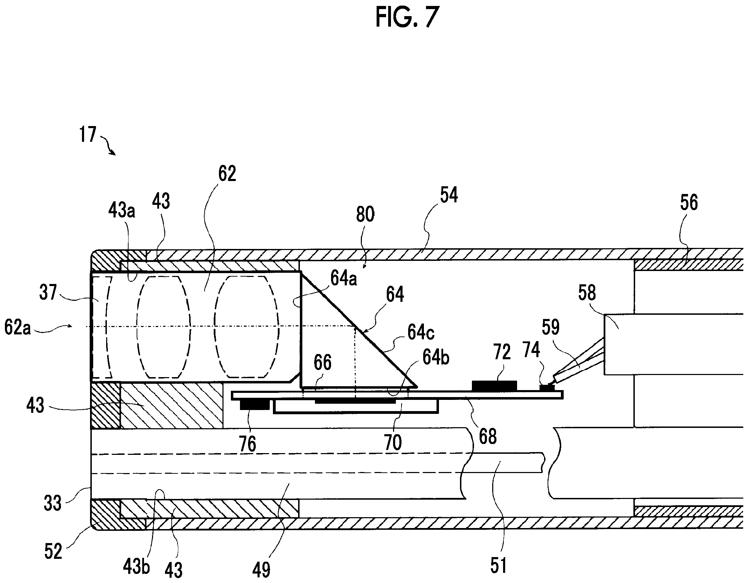

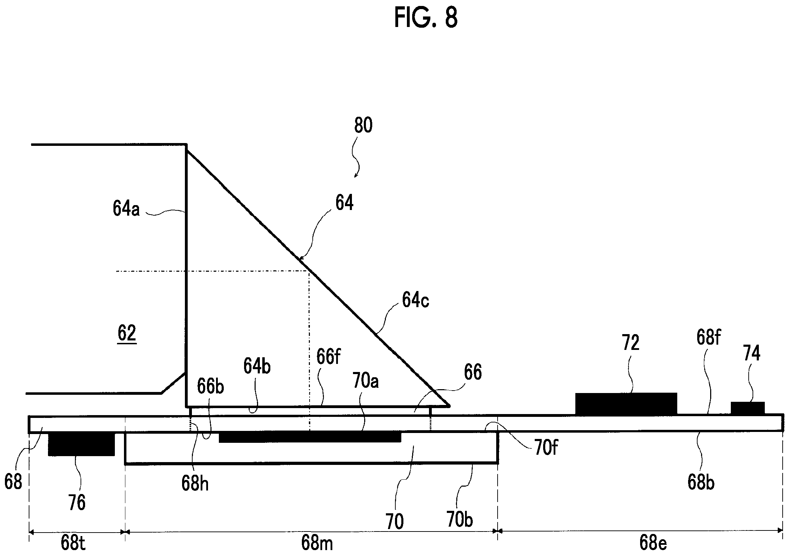

Next, an imaging device, which is an endoscopic imaging device of a second embodiment, will be described. Fig. 7 is a view showing the structure of the imaging device 80 of the second embodiment in the section taken along line A-A of Fig. 2, and Fig. 8 is a view showing only components of the imaging device 80 of the second embodiment. Figs. 7 and 8 are views corresponding to Figs. 3 and 4, respectively; and components, which are the same as or similar to the components of the first embodiment shown in Figs. 3 and 4, will be denoted by the same reference numerals and the description thereof will be omitted.

A board 68 of the imaging device 80 of the second embodiment is disposed parallel to an optical axis of an objective optical system 62a as in the case of the board 68 of the first embodiment.

A rectangular through hole 68h is formed in a region, which faces a light-emitting surface 64b of a rectangular prism 64, of the board 68 of the imaging device 80 of the second embodiment. A rectangular plate-shaped cover glass 66, which is installed on an imaging surface 70a of an imaging element 70, is fitted into the through hole 68h from a back 68b of the board 68, so that the imaging element 70 is mounted on the back 68b of the board 68. Accordingly, image light, which passes through the objective optical system 62a and is reflected by the rectangular prism 64, passes through the cover glass 66 fitted to the through hole 68h and is incident on the imaging surface 70a of the imaging element 70, and the light image of a portion to be observed, which is formed on the imaging surface 70a, is taken by the imaging element 70.

Further, the cover glass 66 is installed on not the entire surface 70f, on which the imaging surface 70a is disposed, of the imaging element 70 of the imaging device 80 of the second embodiment, and is installed in a region of the surface 70f that is limited to the imaging surface 70a and the peripheral portion of the imaging surface 70a, that is, a region (a cover glass-installation region) of the surface 70f that is limited to a part of the surface of the imaging element 70. The imaging element 70 is fixed in a state in which a region (non-cover glass-installation region) of the surface 70f of the imaging element 70 except for the cover glass-installation region is in contact with the back 68b of the board 68.

Further, various connection terminals (electrode pads) are provided in the non-cover glass-installation region of the surface 70f of the imaging element 70, and these connection terminals are electrically connected to connection terminals that are provided on the back 68b of the board 68.

Furthermore, as in the case of the board 68 of the imaging device 60 of the first embodiment, the board 68 of the imaging device 80 of the second embodiment includes a base end-side region 68e that extends toward a base end side from an imaging element mounting region 68m on which the imaging element 70 is mounted, and a tip-side region 68t that extends toward a tip side from the imaging element mounting region 68m.

As in the case of the imaging device 60 of the first embodiment, a drive circuit 76, which is necessary to drive the imaging element 70, is disposed in the tip-side region 68t of the board 68 and an electronic component of the drive circuit 76 is mounted in the tip-side region 68t.

Here, since the imaging element 70 is mounted on the back 68b of the board 68, a gap between the tip-side region 68t of the board 68 and the lens barrel 62 is narrower than that of the imaging device 60 of the first embodiment and a gap enough to mount the electronic component of the drive circuit 76 is not present. Further, it is preferable that the drive circuit 76 is disposed at a position as close to the imaging element 70 as possible. For this reason, the electronic component of the drive circuit 76 is mounted on the back 68b of the board 68.

A peripheral circuit 72 other than the drive circuit 76, which is mounted in the tip-side region 68t, and input and output terminals 74 are disposed in the base end-side region 68e of the board 68 as in the case of the imaging device 60 of the first embodiment, and an electronic component of the peripheral circuit 72 is mounted on the surface 68f of the board 68. The electronic component of the peripheral circuit 72 and the input and output terminals 74 may be disposed on the back 68b without being disposed on the surface 68f of the board 68.

According to the imaging device 80 of the above-mentioned second embodiment, since the drive circuit 76 is disposed in the tip-side region 68t of the board 68 as in the imaging device 60 of the first embodiment, it is possible to reduce the size of the base end-side region 68e to that extent. The length of the imaging device 80 in the direction parallel to the optical axis of the objective optical system 62a can be reduced. Accordingly, it is possible to reduce the size of the endoscope tip portion 17, particularly, to reduce the length of the endoscope tip portion 17 in the axial direction.

Further, since the board 68 is disposed on the surface 70f of the imaging element 70, the board 68 can be disposed closer to the lens barrel 62 than that of the imaging device 60 of the first embodiment. Accordingly, it is possible to reduce the size of a dead space between the board 68 and the lens barrel 62. Therefore, the size of the endoscopic imaging device in the radial direction orthogonal to the optical axis of the objective optical system 62a can be reduced and the diameter of the endoscope tip portion 17 can also be reduced.

The solid-state imaging element 70 and the board 68 have been disposed parallel to the optical axis of the objective optical system 62a in the imaging devices 60 and 80 of the first and second embodiments, but the invention is not limited thereto. For example, a reflector that changes the direction of a light path of the image light, which has passed through the objective optical system 62a, to one direction other than a direction orthogonal to the direction of the light path may be used instead of the rectangular prism 64. In that case, the imaging element 70 and the board 68 may be disposed along a direction orthogonal to the light path bent by the reflector. At this time, the imaging element 70 and the board 68 are disposed along a direction that is not parallel to the objective optical system 62a.

Furthermore, the board 68 has been flat as a whole in the imaging devices 60 and 80 of the first and second embodiments, but may have a bent (curved) portion.

Moreover, the imaging devices 60 and 80 of the first and second embodiments can be used as an imaging device disposed in an imaging section, which takes an image of a portion to be observed, of arbitrary type of endoscope, such as a rigid scope or a capsule type endoscope other than a flexible scope. Even in this case, it is possible to contribute to the reduction of the size of the imaging section of the endoscope.

### Explanation of References

- 11:: body operation unit
- 13:: endoscope insertion part
- 15:: universal cord
- 17:: endoscope tip portion
- 19:: soft portion
- 21:: bendable portion
- 35:: end face
- 37:: observation window
- 43:: tip portion body
- 43a, 43b:: hole
- 49:: forceps pipe
- 51:: air supply/water supply pipe
- 52:: tip cover
- 54:: outer tube
- 56:: nodal ring
- 58:: signal cable
- 59:: signal wire
- 60, 80, 90:: imaging device
- 62:: lens barrel
- 62a:: objective optical system
- 64:: rectangular prism
- 64a:: incident surface
- 64b:: light-emitting surface
- 64c:: inclined surface
- 66:: cover glass
- 66f, 68f, 70f:: surface
- 68:: board
- 68b, 70b:: back
- 68e:: base end-side region
- 68h:: through hole
- 68m:: imaging element mounting region
- 68t:: tip-side region
- 70:: solid-state imaging element (imaging element)
- 70a:: imaging surface
- 72:: peripheral circuit
- 74:: input and output terminals
- 76:: drive circuit
- 100:: endoscope

## Claims

1. An endoscopic imaging device comprising:
an objective optical system that receives image light of a portion to be observed and forms an image;
a reflector changing a direction of a light path of image light, which has passed through the objective optical system, to one direction;
a solid-state imaging element of which an imaging surface is disposed along a plane orthogonal to the direction of the light path changed by the reflector and which takes a light image formed on the imaging surface; and
a board where the solid-state imaging element and an electronic component necessary to drive the solid-state imaging element are mounted and the electronic component is mounted in a region extending toward the objective optical system from a region in which the solid-state imaging element is mounted.

2. The endoscopic imaging device according to claim 1,
wherein the reflector changes the direction of the light path of the image light, which has passed through the objective optical system, at a right angle, and
the imaging surface is disposed parallel to an optical axis of the objective optical system.

3. The endoscopic imaging device according to claim 1 or 2,
wherein the solid-state imaging element and the electronic component are mounted on the same surface of the board.

4. The endoscopic imaging device according to any one of claims 1 to 3,
wherein a region of the board in which the solid-state imaging element is mounted, and a region of the board in which the electronic component is mounted are provided in an area of a flat plate-shaped region of the board that is parallel to the imaging surface.

5. The endoscopic imaging device according to any one of claims 1 to 4,
wherein the objective optical system includes a plurality of lenses, and the plurality of lenses are housed and held in a lens barrel, and
the electronic component mounted on the board is disposed in a gap between the lens barrel and the board.

6. The endoscopic imaging device according to claim 5,
wherein the reflector is a rectangular prism, one surface of two surfaces of the rectangular prism between which a right angle is formed is an incident surface, and the other surface thereof is a light-emitting surface, and the image light, which is emitted from the objective optical system and incident from the incident surface, is reflected by an inclined surface of the rectangular prism and emitted from the light-emitting surface,
the solid-state imaging element is provided with a cover glass that covers the imaging surface, and the light-emitting surface of the rectangular prism is joined to a part of a region of a surface of the cover glass, and
the electronic component is disposed by using the gap that is formed so as to correspond to a thickness of the cover glass and the solid-state imaging element in a region protruding toward the objective optical system from the region, to which the light-emitting surface of the rectangular prism is joined, of the surface of the cover glass.

7. The endoscopic imaging device according to any one of claims 1 to 6,
wherein the solid-state imaging element is an MOS type imaging element, and
the electronic component is a vibrator or an oscillator that generates a clock signal of the solid-state imaging element.

8. The endoscopic imaging device according to any one of claims 1 to 7,
wherein the objective optical system, the reflector, the solid-state imaging element, and the board are mounted in a tip portion of an endoscope insertion part to be inserted into a subject.
